# EUROPEAN PATENT SPECIFICATION

(11) **EP 1 004 292 B1**
(45) Date of publication and mention of the grant of the patent: **13.08.2003**
(21) Application number: 98830702.1
(22) Date of filing: 23.11.1998
(51) Int. Cl.: A61K 9/00

(54) **Method for preparing an effervescent granulate**
Verfahren zur Herstellung eines Brausegranulats
Procédé pour la preparation d'un granule effervescent

(43) Date of publication of application: 31.05.2000
(73) Proprietor: E-PHARMA TRENTO S.p.A., Frazione Ravina - 38040 Trento (IT)
(72) Inventor: Andreatta, Paolo, 38050 Villazzano (Trento) (IT)
(74) Representative: Marchi, Massimo, Dr.

(56) References cited:
- EP-A- 0 624 364
- EP-A- 0 670 160
- EP-A- 0 673 644
- WO-A-95/07070

## Description

The present invention relates to a method for preparing an effervescent granulate, to the granulate thus obtained and to the pharmaceutical forms containing it.

More particularly, the present invention relates to an improvement to the method for preparing effervescent granulates described in EP-A-0 673 644.

Specifically, the method described in the above-mentioned document comprises the phases of a) feeding a fluid bed with a flow of air which has a moisture content of between 0 and 1.0 g/kg, b) spraying water or an aqueous solution onto the mixture of acid and basic ingredients forming the effervescent system in order to carry out the granulation at a temperature of between 30 and 90°C, the sprayed water being evaporated from the particles being granulated before an effervescence reaction takes place, but after a granulation action, c) continuing the said spraying and evaporation until the desired granule sizes are reached, and d) drying the granules thus obtained to a moisture content of between 0 and 1% by weight.

However, it has now been found that keeping the temperature essentially constant during the granulation phase is of prime importance in the said method.

More particularly, the inventors of the present invention have observed that, once the appropriate granulation temperature has been established, and this can vary from one case to another as a function of the initial composition which needs to be granulated, if the temperature increases by a value of more than about 2°C, non-optimal aggregation of the particles takes place and the granular material does not form (Comparative Example 2), whereas, if the temperature falls by a value of more than about 2°C, sudden packing of the composition to be granulated takes place, the latter assuming the shape of a spongy mass which can no longer be processed further (Comparative Example 1).

WO-A-95 07070 relates an effervescent granulated material for a pharmaceutical preparation containing calcium carbonate and citric acid wherein 5-20 parts by weight of the total acid provided for the reaction with the calcium carbonate is replaced by at least one of malic acid, gluconic acid, lactic acid, and their salts.

EP-A-0 624 364 relates to a pharmaceutical formulation of at least one alkali-sensitive active substance with an effervescent system, the carbonate component is embedded in at least one edible, organic acid and is preferably covered by said acid or by another acid. More particularly, the active substance is embedded in at least one of the following compounds: an edible, organic acid, a higher alcohol, a hydrocolloid or a relatively long-chain polyvinylpyrrolidone, preferably covered with at least one of the stated compounds. The contact zone between active substance and effervescent system should have a pH of not more than 4.5. Both effervescent system particles and active substance particles, which are embedded or optionally covered in this manner, may be applied to carrier crystals of the same or another acid. The acid provided for the embedding or covering may contain 0.1 to 3 mg of ethylenediaminetetraacetic acid per tablet.

EP-A-0 670 160 relates to a granular product with an effervescent system which comprises acid-sensitive pharmaceutically active substances, such as, for example, beta-carotene, cimetidine, ranitidine or cisapride, which is specially useful to prevent antacid action, having an acid-binding capacity below about 5meq, at a weight of about 1.6 to about 2.3 grams. The effervescent grains are made from carrier crystals of at least one solid, edible organic acid, preferably citric acid, and are present as a granular product, separate from the pharmaceutically active substance, and are coated with at least one layer of a water-soluble neutral substance which is able to bring about a melting point depression of the acid grains at their surface, such as, for example, a water-soluble polymer, a higher alcohol, a carbohydrate and/or a hydrocolloid. A second coating contains at least a part of the alkali and/or alkaline earth carbonate or bicarbonate provided for the total dosage.

The present invention thus relates to a method for preparing an effervescent granulate according to claim 1.

In the course of the present description and in the claims which follow, the expression "essentially constant" means that the predetermined temperature is kept within a range of about ± 2°C.

The expression "desired characteristics" of the granulate means a residual moisture content of less than 0.5%; an average particle size of between 200 and 400 µ, with not more than 15% of the particles less than 75 µ in size and not more than 15% of the particles greater than 500 µ in size; a flow time for the granulate (100 g) to flow out of a glass funnel (walls inclined at 60° and outlet diameter of 9 mm) of less than 15 sec., preferably less than 10 sec., a tangent of the angle of rest of less than 1; a Carr index of less than 20; and an apparent specific weight of less than 1.0 g/ml.

The expression "aqueous solution" also refers to emulsions.

Examples of suitable acid ingredients which form the effervescent system are organic mono-, di- and tricarboxylic acids and oxyacids, salts of organic acids and salts of inorganic acids.

Typical examples of monocarboxylic acids are lactic acid and ascorbic acids.

Typical examples of dicarboxylic acids are tartaric acid, fumaric acid, malic acid, succinic acid, adipic acid and maleic acid.

A typical example of a tricarboxylic acid is citric acid.

A typical example of a salt of an organic acid is monosodium citrate.

A typical example of a salt of an inorganic acid is sodium hydrogen phosphate.

Examples of suitable basic ingredients in the above-mentioned effervescent system are alkali metal or alkaline-earth metal carbonates and bicarbonates and mixtures thereof.

Typical examples of the said basic ingredients are sodium carbonate, magnesium carbonate, sodium bicarbonate, potassium carbonate, potassium bicarbonate, calcium carbonate and magnesium carbonate.

Preferably, the air flow in phase I is greater than 1000 m³/hour per 100 kg of mixture to be granulated. Even more preferably, the said air flow is greater than 2500 m³/hour.

Generally, the air temperature in phase I is between 40 and 80°C, its value being determined on a case by case basis as a function of the preselected granulation temperature. Advantageously, the air temperature is about 10 - 20°C higher than the granulation temperature. In other words, the air temperature is about 10 - 20°C higher than the temperature of the mixture subjected to granulation.

The moisture content of the air introduced into the granulator is preferably less than 2 g/kg, even more preferably less than 1 g/kg.

Typically, the temperature of the said mixture during the granulation is between 30 and 60°C. Preferably, it is between 30 and 40°C.

When the granulation is carried out by spraying an aqueous solution, the solute in the said solution is chosen from the group comprising diluents such as, for example, sorbitol, mannitol, lactose, sucrose, glucose and fructose, binders such as, for example, polyvinylpyrrolidone and sorbitol, sweeteners such as, for example, sodium saccharin, aspartame, acesulfame K, sodium cyclamate and sucrose, anti-foaming agents such as, for example, dimethyl polysiloxane, wetting agents such as, for example, sucrose monopalmitate, sodium lauryl sulphate, sodium docusate, sorbitan esters, sodium hexametaphosphate, organic and inorganic dyes, and mixtures thereof.

Preferred examples of the said solutions are those comprising sodium saccharin, sodium docusate, sorbitol, sucrose monopalmitate, polyvinylpyrrolidone, Sunset yellow and mixtures thereof.

Preferably, the temperature of the water or of the solution sprayed is between 30 and 50°C.

The flow of water or of the spray solution is preferably between 100 and 2000 ml/min, even more preferably between 500 and 1500 ml/min. In turn, the said flow preferably has a pressure of between 2 and 4 atmospheres.

The amount of water used for the granulation is preferably between 2 and 10% by weight relative to the mass to be granulated.

Generally, the effervescent granulate prepared as described above is then mixed in a rotary mixer with pharmacologically active ingredients and other conventional and suitable inactive ingredients in order to prepare effervescent pharmaceutical forms typically in the form of granulates or tablets.

Typical characteristics of the mixture thus obtained are as follows (before any tableting):
residual moisture content of less than 1.0%;
time taken for the granulate to flow out (fluidity) of a glass funnel (walls inclined at 60° and outlet diameter of 9 mm) of less than 15 sec.;
tangent of the angle of rest of less than 1;
Carr index of less than 20;
and force of expulsion of the tablets from the matrix of less than 400 N, which indicates good lubrication.

In an alternative embodiment of the present invention, the pharmacologically active ingredients which are stable up to at least 50°C and which are non-oxidizable in air, such as, for example, paracetamol, can be added to the mixture comprising at least one acid ingredient and one basic ingredient even before the granulation process is started.

It has moreover been found that, when the acid ingredient used to form the effervescent granulate is citric acid, it is necessary to use a first portion thereof which has a particle size of less than 100 µ (which is referred to hereinbelow as "citric acid in powder form") and a second portion which has a particle size of between 200 and 400 µ (which is referred to hereinbelow as "citric acid in crystalline form").

The reason for this is that the inventors of the present invention have observed that when the citric acid used is all in powder form, a non-flowing granulate is obtained. Moreover, when the said granulate is used to prepare tablets, a high average tableting force (90 KN) is required and, during the phase of expulsion of the tablet from the matrix, the phenomenon of capping (Comparative Example 4) is observed. On the other hand, when the citric acid used is all in crystalline form, a granulate is obtained which cannot be tableted, even under an average force of greater than 90 KN (Comparative Example 3).

Preferably, the portion of citric acid in powder form is equal to 40-60% by weight.

When the pharmaceutical form of the present invention is in tablet form, the punch and the matrix used are preferably chosen from those capable of making suitable splitting incisions on the tablet, such as, for example, a median incision, which makes it easier to divide the tablet into two parts.

Besides the usual excipients, the said pharmaceutical form can also contain other conventional ingredients, for instance preserving agents such as, for example, ascorbic acid, sodium benzoate and para-oxybenzoates, lubricants such as, for example, polyethylene glycols (PEGs) with a molecular weight of between 4000 and 8000, adipic acid, L-leucine and sodium benzoate, flavourings and dyes.

The following examples illustrate the present invention.

### EXAMPLE 1

### a) Preparation of the effervescent granulate

A mixture formed of anhydrous citric acid in crystalline form (65 kg), anhydrous citric acid in powder form (65 kg), sodium bicarbonate (50 kg), anhydrous sodium carbonate (20 kg) and sorbitol in powder form (87 kg) was loaded into a fluid-bed granulator (volume: 1200 litres). This mixture was heated to 40°C and kept in suspension by means of a stream of dried air (R.H. < 1.0 g/kg, air temperature of 60°C; air throughput = 3000 m³/hour).

The said mixture was then granulated by spraying an aqueous solution, at 40°C, formed of an emulsion comprising dimethicone (0.5 kg), sodium saccharin (2.5 kg) and purified water (14.5 kg). The spraying was carried out by passing the aqueous solution (throughput of 1 litre/min) through spray nozzles (1.0 mm in diameter), at a pressure of 3 atmospheres. The granulation was continued until the temperature of the mixture had fallen to 38°C, and the flow of the sprayed granulating aqueous solution was interrupted until the mixture had again reached a temperature of 40°C. The granulation was continued in this way until all of the granulating aqueous solution had been used.

The granulate was dried by means of a flow of air heated to 60°C, until the temperature of the air exiting had reached the temperature of the air entering (60°C). The granules thus obtained were then cooled and removed from the granulator under an atmosphere with an R.H. ≤ 20 g/kg and temperature of 18-22°C.

The granulate thus obtained had the following characteristics:

| | |
|---|---|
| Residual moisture content | 0.2% |
| Average particle size | 300 µ |
| Fluidity^{(*)} | 6 sec. |

| | |
|---|---|
| ^{(*)} The fluidity was measured as the time taken for the granulate (100 g) to flow out of a glass funnel with walls inclined at 60° and with an outlet diameter of 9 mm. | |

| | |
|---|---|
| Tangent of the angle of rest | 0.6 |
| Carr index | 13 |
| Apparent specific weight | 0.65 g/ml |

Lastly, the granulate was stabilized by a flow of carbon dioxide (30 litres/hour) for 12 hours.

### b) Preparation of the mixture before tableting

Ascorbic acid (5 kg), as active ingredient, and a flavouring (5 kg) were first added to the granulate (294.5 kg) obtained as described in the above-mentioned point a). This mixture was kept stirring until a uniform mixture was obtained. Next, PEG 6000 (5 kg) was added and stirring was continued for a further 5 minutes.

### c) Tableting

The above-mentioned mixture was tableted using a force of 40 KN to form tablets of 4 g each, comprising 1.0 g of ascorbic acid each. The said tablets were packaged in premises with an R.H. ≤ 20 g/kg and at a temperature of 18-22°C.

### EXAMPLE 2

### a) Preparation of the effervescent granulate

A mixture formed of paracetamol (50 kg), as active ingredient, anhydrous citric acid in crystalline form (50 kg), anhydrous citric acid in powder form (50 kg), sodium bicarbonate (80 kg), anhydrous sodium carbonate (20 kg) and sorbitol in powder form (35 kg) was loaded into a fluid-bed granulator (volume: 1200 litres). This mixture was heated to 35°C and kept in suspension by means of a stream of dried air (R.H. < 1.0 g/kg, air temperature of 55°C; air throughput = 3000 m³/hour). The said mixture was then granulated by spraying an aqueous solution, at 35°C, formed of sorbitol (4.5 kg), sodium saccharin (2.0 kg), sodium docusate (0.5 kg) and purified water (10 kg). The spraying was carried out by passing the aqueous solution (throughput of 1 litre/min) through spray nozzles (1.2 mm in diameter), at a pressure of 3 atmospheres. The granulation was continued until the temperature of the powder had fallen to 33°C, and the flow of the sprayed granulating aqueous solution was interrupted until the mixture had again reached a temperature of 35°C. The granulation was continued in this way until all of the granulating aqueous solution had been used.

The granulate was dried by means of a flow of air heated to 55°C, until the temperature of the air exiting had reached the temperature of the air entering (55°C). The granules thus obtained were then cooled and removed from the granulator under an atmosphere with an R.H. ≤ 20 g/kg and temperature of 18-22°C.

The granulate thus obtained had the following characteristics:

| | |
|---|---|
| Residual moisture content | 0.3% |
| Average particle size | 350 µ |
| Fluidity | 8 sec. |
| Tangent of the angle of rest | 0.65 |
| Carr index | 14 |
| Apparent specific weight | 0.760 g/ml |

Lastly, the granulate was stabilized by a flow of carbon dioxide (30 litres/hour) for 12 hours.

### b) Preparation of the mixture before tableting

Flavouring (3 kg) was first added to the granulate (297.4 kg) obtained as described in the above-mentioned point a). This mixture was kept stirring until a uniform mixture was obtained. Next, PEG 6000 (5 kg) was added and stirring was continued for a further 5 minutes.

### c) Tableting

The above-mentioned mixture was tableted using a force of 60 KN to form tablets of 3 g each, comprising 0.5 g of paracetamol each. The said tablets were packaged in premises with an R.H. ≤ 20 g/kg and at a temperature of 18-22°C.

### EXAMPLE 3

### a) Preparation of the effervescent granulate

A mixture formed of anhydrous citric acid in crystalline form (84 kg), anhydrous citric acid in powder form (36 kg), sodium bicarbonate (59.8 kg), anhydrous potassium carbonate (15 kg) and sorbitol in powder form (90 kg) was loaded into a fluid-bed granulator (volume: 1200 litres). This mixture to be granulated was heated to 40°C and kept in suspension by means of a stream of dried air (R.H. < 1.0 g/kg, air temperature of 60°C; air throughput = 3000 m³/hour).

The said mixture was then granulated by spraying an aqueous solution, at 40°C, formed of tartrazine (0.15 kg), as artificial dye, sodium saccharin (2.0 kg) and purified water (10 kg). The spraying was carried out by passing the aqueous solution (throughput of 1 litre/min) through spray nozzles (1.0 mm in diameter), at a pressure of 3 atmospheres. The granulation was continued until the temperature of the powder had fallen to 38°C, and the flow of the sprayed granulating aqueous solution was interrupted until the mixture had again reached a temperature of 40°C. The granulation was continued in this way until all of the granulating aqueous solution had been used.

The granulate was dried by means of a flow of air heated to 60°C, until the temperature of the air exiting had reached the temperature of the air entering (60°C). The granules thus obtained were then cooled and removed from the granulator under an atmosphere with an R.H. ≤ 20 g/kg and temperature of 18-22°C.

The granulate thus obtained had the following characteristics:

| | |
|---|---|
| Residual moisture content | 0.18% |
| Average particle size | 340 µ |
| Fluidity | 7 sec. |
| Tangent of the angle of rest | 0.55 |
| Carr index | 12 |
| Apparent specific weight | 0.78 g/ml |

Lastly, the granulate was stabilized by a flow of carbon dioxide (30 litres/hour) for 12 hours.

### b) Preparation of the mixture before tableting

Ascorbic acid (100 kg), flavouring (5 kg) and beetroot red (3 kg) as dye were first added to the granulate (286.95 kg) obtained as described in the above-mentioned point a). This mixture was kept stirring until a uniform mixture was obtained. Next, leucine (5 kg) in powder form was added and stirring was continued for a further 5 minutes.

### c) Tableting

The above-mentioned mixture was tableted using an average force of 40 KN to form tablets of 4 g each, comprising 1.0 g of ascorbic acid each. The said tablets were packaged in premises with an R.H. ≤ 20 g/kg and at a temperature of 18-22°C.

### EXAMPLE 4

### a) Preparation of the effervescent granulate

A mixture formed of paracetamol (50 kg), as active ingredient, anhydrous citric acid in crystalline form (50 kg), anhydrous citric acid in powder form (57.8 kg), sodium bicarbonate (120 kg), sodium saccharin (4.0 kg) and sorbitol in powder form (54 kg) was loaded into a fluid-bed granulator (volume: 1200 litres). This mixture was heated to 35°C and kept in suspension by means of a stream of dried air (R.H. < 1.0 g/kg, air temperature of 55°C; air throughput = 3000 m³/hour). The said mixture was then granulated by spraying an aqueous solution, at 35°C, formed of sucrose monopalmitate (0.1 kg), polyvinylpyrrolidone (0.1 kg) and purified water (10 kg). The spraying was carried out by passing the aqueous solution (throughput of 1 litre/min) through spray nozzles (1.2 mm in diameter), at a pressure of 3 atmospheres.

The granulation was continued until the temperature of the powder had fallen to 33°C, and the flow of the sprayed granulating aqueous solution was interrupted until the mixture had again reached a temperature of 35°C. The granulation was continued in this way until all of the granulating aqueous solution had been used.

The granulate was dried by means of a flow of air at 55°C, until the temperature of the air exiting had reached the temperature of the air entering (55°C). The granules thus obtained were then cooled and removed from the granulator under an atmosphere with an R.H. ≤ 20 g/kg and temperature of 18-22°C.

The granulate thus obtained had the following characteristics:

| | |
|---|---|
| Residual moisture content | 0.32% |
| Average particle size | 360 µ |
| Fluidity | 7 sec. |
| Tangent of the angle of rest | 0.62 |
| Carr index | 16 |
| Apparent specific weight | 0.66 g/ml |

The granulate was stabilized by a flow of carbon dioxide (30 litres/hour) for 12 hours.

### b) Preparation of the mixture before tableting

Flavouring (4 kg) was first added to the granulate (336 kg) obtained as described in the above-mentioned point a). This mixture was kept stirring until a uniform mixture was obtained.

### c) Tableting

The above-mentioned mixture was tableted using an average force of 50 KN to form tablets of 3 g each, comprising 0.5 g of paracetamol each. The said tablets were packaged in premises with an R.H. ≤ 20 g/kg and at a temperature of 18-22°C.

### EXAMPLE 5

### a) Preparation of the effervescent granulate

A mixture formed of calcium carbonate (125 kg), as active ingredient, anhydrous citric acid in crystalline form (120 kg), anhydrous citric acid in powder form (80 kg), sodium bicarbonate (80 kg), anhydrous sodium carbonate (20 kg), sorbitol in powder form (7.8 kg) and mannitol (3.5 kg) was loaded into a fluid-bed granulator (volume: 1200 litres). This mixture to be granulated was heated to 45°C and kept in suspension by means of a stream of dried air (R.H. < 1.0 g/kg, air temperature of 70°C; air throughput = 3500 m³/hour).

The said mixture was then granulated by spraying an aqueous solution, at 45°C, formed of sodium saccharin (2.0 kg), polyvinylpyrrolidone (0.025 kg), Sunset yellow (0.025 kg) as dye and purified water (20 kg) at a temperature of 45°. The spraying was carried out by passing the aqueous solution (throughput of 0.5 litre/min) through spray nozzles (1.0 mm in diameter), at a pressure of 3 atmospheres. The granulation was continued until the temperature of the powder had fallen to 43°C, and the flow of the sprayed granulating aqueous solution was interrupted until the mixture had again reached a temperature of 45°C. The granulation was continued in this way until all of the granulating aqueous solution had been used.

The granulate was dried by means of a flow of air heated to 70°C, until the temperature of the air exiting had reached the temperature of the air entering (70°C). The granules thus obtained were then cooled and removed from the granulator under an atmosphere with an R.H. ≤ 20 g/kg and temperature of 18-22°C.

The granulate thus obtained had the following characteristics:

| | |
|---|---|
| Residual moisture content | 0.4% |
| Average particle size | 320 µ |
| Fluidity | 9 sec. |
| Tangent of the angle of rest | 0.65 |
| Carr index | 18 |
| Apparent specific weight | 0.59 g/ml |

Lastly, the granulate was stabilized by a flow of carbon dioxide (30 litres/hour) for 12 hours.

### b) Preparation of the effervescent tablet

Flavouring (4 kg) was first added to the granulate (338.5 kg) obtained as described in the above-mentioned point a). This mixture was kept stirring until a uniform mixture was obtained. Next, PEG 6000 (7.5 kg) was added and stirring was continued for a further 5 minutes.

### c) Tableting

The above-mentioned mixture was tableted using an average force of 50 KN to form tablets of 7 g each, corresponding 2.5 g of calcium carbonate each. The said tablets were packaged in premises with an R.H. ≤ 20 g/kg and at a temperature of 18-22°C.

### COMPARATIVE EXAMPLE 1

The process was performed as in Example 3a) above, except that the flow of the sprayed granulating aqueous solution was not interrupted and the temperature fell gradually. Before the granulating solution had been used up, a sudden packing of the mass to be granulated was observed at 25°C. The product was thus removed from the granulator in the form of a spongy mass and it was not possible to continue the granulation.

### COMPARATIVE EXAMPLE 2

The process was performed as in Example 3a) above, except that the granulation temperature was left to increase gradually up to 60°C. At 55°C, no further aggregation of the particles was observed.

### COMPARATIVE EXAMPLE 3

The process was performed as in Example 4a) and b) above, except that a mixture to be granulated formed of paracetamol (50 kg), as active ingredient, anhydrous citric acid in crystalline form (107.8 kg), sodium bicarbonate (120 kg), sodium saccharin (4 kg), sorbitol in powder form (54 kg), sucrose monopalmitate (0.1 kg) and polyvinylpyrrolidone (0.1 kg) was loaded into the granulator and the mixture was then granulated by spraying with purified water (10 kg).

The granulate thus obtained had the same characteristics as the one obtained according to Example 4 above, apart from a higher average particle size (400-600 µ).

The mixture prepared as described in Example 4b) above could not be tableted even with an average tableting force of greater than 90 KN.

### COMPARATIVE EXAMPLE 4

### a) Preparation of the effervescent granulate

The process was performed as in Example 5a) above, except that a mixture to be granulated formed of calcium carbonate (125 kg), as active ingredient, anhydrous citric acid in powder form (200 kg), sorbitol powder (7.8 kg), mannitol powder (3.5 kg), sodium saccharin (2.0 kg), polyvinylpyrrolidone (0.1 kg) and Sunset yellow dye (0.025 kg) was loaded into the granulator and the mixture was then granulated by spraying with purified water (20 kg).

The granulate thus obtained had the following characteristics:

| | |
|---|---|
| Residual moisture content | 0.4% |
| Average particle size | 120 µ |
| Fluidity | does not flow |
| Tangent of the angle of rest | 1.1 |
| Carr index | 32 |
| Apparent specific weight | 0.40 g/ml |

### b) Preparation of the mixture before tableting

The process was performed as described in Example 5b) above.

The mixture thus obtained had the following characteristics:

| | |
|---|---|
| Residual moisture content | 0.6% |
| Fluidity | does not flow |
| Tangent of the angle of rest | 1.1 |
| Carr index | 31 |

### c) Tableting

The above-mentioned mixture was tableted with a force of 90 KN to form tablets of 7.0 g each, comprising 2.5 g of calcium carbonate each. These tablets, with a hardness of greater than 70 N each, displayed the phenomenon of capping during the phase of expulsion of the tablets from the matrix.

## Claims

1. Method for preparing an effervescent granulate from a mixture comprising at least one acid ingredient and at least one basic ingredient, which are suitable for forming an effervescent system, in which the said mixture is granulated with water in a fluid air bed granulator, by means of the following phases:
I. feeding the said granulator with a flow of air heated to a predetermined temperature and having a moisture content of between 0 and 2 g/kg;
II. wetting the said mixture, comprising at least one acid ingredient and at least one basic ingredient, with sprayed water or a sprayed aqueous solution or a sprayed emulsion,
III. evaporating the sprayed water from the said mixture; and
IV. continuing phases II and III until it is formed a granulate having a residual moisture content of less than 0.5%, an average particle size of between 200 and 400 µ, with not more than 15% of the particles less than 75 µ in size and not more than 15% of the particles greater than 500 µ in size; a flow time for the granulate (100g) to flow out of a glass funnel (walls inclined at 60°C and outlet diameter of 9 mm) of less than 15 sec, preferably less than 10 sec, a tangent of the angle of rest of less than 1; a Carr index of less than 20; and an apparent specific weight of less than 1.0 g/ml.
**characterized in that**
phases II, III and IV are carried out at a temperature which is predetermined as a function of the composition to be granulated, and **in that** the said temperature is kept within a range of ± 2°C.

2. Method according to claim 1, **characterized in that**, in phase I, the said air flow is greater than 1000 m³/hour per 100 kg of mixture to be granulated.

3. Method according to either of the preceding claims 1 and 2, **characterized in that**, in phase I, the air temperature is between 40 and 80°C.

4. Method according to any one of the preceding claims 1 to 3, **characterized in that** the moisture content of the air introduced into the granulator is less than 2 g/kg.

5. Method according to any one of the preceding claims 1 to 4, **characterized in that** the temperature of the said mixture during the granulation is between 30 and 60°C.

6. Method according to any one of the preceding claims 1 to 5, **characterized in that** the temperature of the water or of the solution sprayed is between 30 and 50°C.

7. Method according to any one of the preceding claims 1 to 6, **characterized in that** the said flow of water or of spray solution is between 100 and 2000 ml/min.

8. Method according to any one of the preceding claims 1 to 7, **characterized in that** the said flow of water or of spray solution has a pressure of between 2 and 4 atmospheres.

9. Method according to any one of the preceding claims 1 to 8, **characterized in that** the amount of water used for the granulation is between 2 and 10% by weight relative to the mass to be granulated.

## Patentansprüche

1. Verfahren zur Herstellung eines Brausepulvergranulats aus einer Mischung, die mindestens einen sauren Bestandteil und mindestens einen basischen Bestandteil, die zur Ausbildung eines Brausepulversystems geeignet sind, umfasst, worin die Mischung mit Wasser in einem Luftfliessbettgranulator mittels der nachfolgenden Phasen granuliert wird:
(I) Zuführen eines auf eine vorherbestimmte Temperatur erwärmten Luftstroms mit einem Feuchtigkeitsgehalt von 0-2 g/kg in den Granulator;
(II) Benetzen der Mischung, die mindestens einen sauren Bestandteil und mindestens einen basischen Bestandteil umfasst, mit Sprühwasser, einer wässrigen Sprühlösung oder einer Sprühemulsion;
(III) Verdampfen des Sprühwassers aus der Mischung; und
(IV) Fortführen der Phasen (II) und (III) bis ein Granulat mit einem Restfeuchtigkeitsgehalt von weniger als 0,5 %, einer durchschnittlichen Teilchengrösse von 200-400 µm mit nicht mehr als 15 % der Teilchen mit einer Grösse von 75 µm und nicht mehr als 15 % der Teilchen mit einer Grösse von mehr als 500 µm, einer Fliesszeit des Granulats (100 g) zum Ausfliessen aus einem Glastrichter (Wände mit einer Neigung von 60° und einem Auslassdurchmesser von 9 mm) von weniger als 15 Sekunden, vorzugsweise weniger als 10 Sekunden, einem Tangens des Haltewinkels von weniger als 1, einem Carr-Index von weniger als 20 und einer Schüttdichte von weniger als 1,0 g/ml gebildet wird,
**dadurch gekennzeichnet, dass** die Phasen (II), (III) und (IV) bei einer Temperatur durchgeführt werden, die als Funktion der zu granulierenden Zusammensetzung vorherbestimmt wird, und dass die Temperatur innerhalb eines Bereichs von ± 2°C gehalten wird.

2. Verfahren gemäss Anspruch 1, **dadurch gekennzeichnet, dass** in Phase (I) der Luftfluss mehr als 1.000 m³/h/100 kg der zu granulierenden Mischung beträgt.

3. Verfahren gemäss mindestens einem der vorhergehenden Ansprüche 1 und 2, **dadurch gekennzeichnet, dass** in Phase (I) die Lufttemperatur zwischen 40 und 80°C liegt.

4. Verfahren gemäss mindestens einem der vorhergehenden Ansprüche 1 bis 3, **dadurch gekennzeichnet, dass** der Feuchtigkeitsgehalt der in den Granulator eingeführten Luft weniger als 2 g/kg beträgt.

5. Verfahren gemäss mindestens einem der vorhergehenden Ansprüche 1 bis 4, **dadurch gekennzeichnet, dass** die Temperatur der Mischung während des Granuliervorgangs zwischen 30 und 60°C liegt.

6. Verfahren gemäss mindestens einem der vorhergehenden Ansprüche 1 bis 5, **dadurch gekennzeichnet, dass** die Temperatur des aufgesprühten Wassers oder der aufgesprühten Lösung zwischen 30 und 50°C liegt.

7. Verfahren gemäss mindestens einem der vorhergehenden Ansprüche 1 bis 6, **dadurch gekennzeichnet, dass** die Flussmenge des Wassers oder der Sprühlösung zwischen 100 und 2.000 ml/min liegt.

8. Verfahren gemäss mindestens einem der vorhergehenden Ansprüche 1 bis 7, **dadurch gekennzeichnet, dass** der Wasser- oder Sprühlösungsfluss unter einem Druck von zwischen 2 und 4 Atm steht.

9. Verfahren gemäss mindestens einem der vorhergehenden Ansprüche 1 bis 8, **dadurch gekennzeichnet, dass** die Menge an Wasser, die für den Granuliervorgang verwendet wird, bezogen auf die zu granulierende Masse, zwischen 2 und 10 Gew.% liegt.

## Revendications

1. Procédé pour la préparation d'un granulat effervescent à partir d'un mélange comprenant au moins un ingrédient acide et au moins un ingrédient basique qui sont convenables pour la formation d'un système effervescent dans lequel ledit mélange est granulé avec de l'eau dans un granulateur en lit fluidisé pneumatique, par le biais des phases suivantes :
I. alimentation dudit granulateur avec un flux d'air chauffé à une température prédéterminée et ayant une teneur en eau entre 0 et 2 g/kg ;
II. humidification dudit mélange, comprenant au moins un ingrédient acide et au moins un ingrédient basique, avec de l'eau pulvérisée ou une solution aqueuse pulvérisée ou une émulsion pulvérisée ;
III. évaporation de l'eau pulvérisée dudit mélange ; et
IV. poursuite des phases II et III jusqu'à ce qu'il se forme un granulat ayant une teneur en eau inférieure à 0,5%, une taille moyenne de particules entre 200 et 400 µm avec pas plus de 15% des particules de taille inférieure à 75 µm et pas plus de 15% des particules de taille supérieure à 500 µm ; une durée d'écoulement pour que le granulat (100g) s'écoule de l'entonnoir en verre (parois inclinées à 60°C et diamètre de sortie de 9 mm) inférieure à 15 s, de préférence inférieure à 10 s, une tangente d'angle de repos inférieure à 1 ; un indice de Carr inférieur à 20 ; et un poids spécifique apparent inférieur à 1,0 g/ml, **caractérisé en ce que** les phases II, III et IV sont menées à une température qui est prédéterminée en fonction de la composition à granuler, et **en ce que** ladite température est maintenue dans un intervalle de ± 2°C.

2. Procédé selon la revendication 1, **caractérisé en ce que**, lors de la phase I, ledit flux d'air est supérieur à 1000 m³/heure pour 100 kg de mélange à granuler.

3. Procédé selon l'une ou l'autre des revendications 1 et 2, **caractérisé en ce que**, lors de la phase I, la température de l'air est entre 40°C et 80°C.

4. Procédé selon l'une quelconque des revendications précédentes 1 à 3, **caractérisé en ce que** la teneur en. eau de l'air introduit dans le granulateur est inférieure à 2 g/kg.

5. Procédé selon l'une quelconque des revendications précédentes 1 à 4, **caractérisé en ce que** la température dudit mélange pendant la granulation est entre 30°C et 60°C.

6. Procédé selon l'une quelconque des revendications précédentes 1 à 5, **caractérisé en ce que** la température de l'eau ou de la solution pulvérisée est entre 30°C et 50°C.

7. Procédé selon l'une quelconque des revendications précédentes 1 à 6, **caractérisé en ce que** ledit flux d'eau ou de solution pulvérisée est entre 100 et 2000 ml/min.

8. Procédé selon l'une quelconque des revendications précédentes 1 à 7, **caractérisé en ce que** ledit flux d'eau ou de solution pulvérisée a une pression entre 2 et 4 atmosphères.

9. Procédé selon l'une quelconque des revendications précédentes 1 à 8, **caractérisé en ce que** la quantité d'eau utilisée pour la granulation est entre 2% et 10% en poids par rapport à la masse à granuler.
